# EUROPEAN PATENT SPECIFICATION

(11) **EP 3 485 892 B1**
(45) Date of publication and mention of the grant of the patent: **13.10.2021**
(21) Application number: 17827719.0
(22) Date of filing: 13.07.2017
(51) Int. Cl.: A61K 33/00, A61P 25/28, A23L 2/52, A23L 33/10, A61K 9/08, A61K 9/16, A61K 9/00

(54) **COMPOSITION FOR PREVENTION OR TREATMENT OF MILD COGNITIVE IMPAIRMENT OR DEMENTIA CONTAINING HYDROGEN AS ACTIVE INGREDIENT**
ZUSAMMENSETZUNG ZUR PRÄVENTION ODER BEHANDLUNG VON LEICHTER KOGNITIVER BEEINTRÄCHTIGUNG ODER DEMENZ MIT WASSERSTOFF ALS WIRKSTOFF
COMPOSITION DESTINÉE À LA PRÉVENTION OU AU TRAITEMENT D'UN DÉFICIT COGNITIF LÉGER OU D'UNE DÉMENCE COMPRENANT DE L'HYDROGÈNE EN TANT QUE PRINCIPE ACTIF

(30) Priority: 13.07.2016 JP 2016138793
(43) Date of publication of application: 22.05.2019
(73) Proprietor: Mitos Co., Ltd., Kawasaki-shi, Kanagawa 211-0005 (JP); Medical Corporation Association Sochikai, Tokyo 113-0034 (JP)
(72) Inventor: ASADA, Takashi, Bunkyo-ku Tokyo 113-0034 (JP); OHTA, Shigeo, Kawasaki-shi Kanagawa 211-0004 (JP)
(74) Representative: Mewburn Ellis LLP
(86) International application number: PCT/JP2017/025575
(87) International publication number: WO 2018/012596

(56) References cited:
- JP-A- 2009 114 084
- JP-A- 2013 146 373
- KAZUFUMI NAGATA ET AL: "Consumption of Molecular Hydrogen Prevents the Stress-Induced Impairments in Hippocampus-Dependent Learning Tasks during Chronic Physical Restraint in Mice", NEUROPSYCHOPHARMACOLOGY., vol. 34, no. 2, 18 June 2008 (2008-06-18), pages 501-508, XP055661494, US ISSN: 0893-133X, DOI: 10.1038/npp.2008.95
- KIYOMI NISHIMAKI ET AL: "Effects of Molecular Hydrogen Assessed by an Animal Model and a Randomized Clinical Study on Mild Cognitive Impairment", CURRENT ALZHEIMER RESEARCH, vol. 15, no. 5, 14 March 2018 (2018-03-14) , pages 482-492, XP55661340, NL ISSN: 1567-2050, DOI: 10.2174/1567205014666171106145017
- SHIGEO OTA: "Suisosui wa Karada ni Yoi ka? Warui ka? Suisosui no Seitai eno Koka", Anti- Aging Medicine, vol. 4, no. 6, 2008, pages 778-784, XP009518290,
- KAZUFUMI NAGATA et al.: "Suisosui ga Kosoku Model Mouse no Ninchi Kino ni Oyobosu Eikyo", Journal of Japanese Biochemical Society, vol. 79, no. 1, 2007, page 3P-1048, XP009513793,
- Ota, Shigeo: "Ninchisho Yobo eno Kenkyu Shokai: Suiso ni yotte Ninchisho o Yobo dekiru ka?", Machigurumi Ninchisho Sodan Center News Letter, vol. 1, 2010, pages 1-4, XP009518794,
- GU, YEUNHWA et al.: "Drinking Hydrogen Water Ameliorated Cognitive Impairment in Senescence- Accelerated Mice", J. Clin. Biochem. Nutr., vol. 46, 2010, pages 269-276, XP055569786,
- LI, JIAN et al.: "Hydrogen-rich saline improves memory function in a rat model of amyloid-be- ta-induced Alzheimer's disease by reduction of oxidative stress", Brain Res., vol. 1328, 2010, pages 152-161, XP026999980,

## Description

### Technical Field

The present invention relates to a composition for prevention or treatment for mild cognitive impairment or dementia.

### Background Art

Mild cognitive impairment (MCI) is a prodromal stage for dementia and it is said that neglect of MCI results in continuous decline of the cognitive function and progression to dementia in approximately 50% of people in 5 years. A risk factor of mild cognitive impairment and Alzheimer-type dementia is the ApoE ε4 genetic polymorphism of the apolipoprotein E gene (see Non-Patent Literature 1). Whereas the 112th amino acid in the ApoE ε2 and ApoE ε3 gene products is cysteine, the 112th amino acid in the ApoE ε4 gene product is arginine. The odds ratio of Alzheimer-type dementia for heterozygous ApoE ε4 gene carriers is 3.2 and the odds ratio for homozygous carriers is 11.6. Simultaneously, the ApoE ε4 genetic polymorphism is a risk factor for arteriosclerosis.

Meanwhile, hydrogen has recently been found to have various effects besides the conventional antioxidant capacity and many additional reports have already been published also on its effect and safety (see Patent Literature 1).

The effect of hydrogen intake on cognitive function in animals has been studied.

Previously it was shown that mice subjected to physical restraint stress displayed impaired learning and memory, increased oxidative stress markers and a reduction in progenitor cell proliferation. Drinking hydrogen water reduced these symptoms in mice in comparison to mice who drank degassed water (Non patent literature 2).

Gu et al showed that consumption of hydrogen water produced using a magnesium stick can reduce age related decline in cognitive ability in a SAMP8 mouse model (Non patent literature 3).

In Li et al, rats were injected with Aβ1-42 to create a rat model of Alzheimer's disease. Aβ1-42 injected rats treated with hydrogen rich saline showed improved learning and memory compared with those treated with physiological saline as determined by the Morris water maze test. Furthermore, long term potentiation was enhanced and levels of inflammatory cytokines were supressed in rats treated with hydrogen rich saline compared to those treated with physiological saline (Non patent literature 4).

Non patent literature 5-7 also investigate the role of hydrogen in suppressing cognitive decline or preventing dementia.

Patent literature 2 discloses a composition comprising hydrogen for use in promoting neurogenesis in mammals to improve cognitive function and also for use as an antidepressant. Patent literature 3 discloses a compact device for generating hydrogen gas for inhalation. The authors note the contribution of reactive oxygen species to various diseases including Alzheimer's disease and the use of hydrogen as an antioxidant to treat these diseases.

### Citation List

### Patent Literature

Patent Literature 1: International Publication No. WO 2007/021034
Patent Literature 2: JP2009114084 A
Patent Literature 3: JP2013146373 A

### Non Patent Literature

Non Patent Literature 1: S. Cosentino et al., Neurology, May 6, 2008 vol. 70 no. 19 Part 2 1842-1849
Non Patent Literature 2: K. Nagata et al., Neuropsychopharmacology, June 16, 2008, vol. 34 no. 2, pg 501-508
Non Patent Literature 3: Y. Gu et al., J. Clin. Biochem. Nutr., 2010, vol 46, pg 269-276 Non
Patent Literature 4: J. Li et al., Brain Res., 2010, vol 1328, pg 152-161
Non Patent Literature 5: S. Ota et al., Anti-Aging Medicine, 2008, vol 4, pg 778-784
Non Patent Literature 6: K. Nagata et al., J. Japanese Biochem. Soc., 2007, vol 79, abstract 3P-1048
Non Patent Literature 7: S. Ota et al., Machigurumi Ninchisho Sodan Center News Letter, 2010, vol 1, pg 3, 5

### Summary of Invention

This invention is defined by the claims. Any subject matter falling outside the scope of the claims is provided for information purposes only. Any references in the description to methods of treatment refer to the compounds, pharmaceutical compositions and medicaments of the present invention for use in a method for treatment of the human or animal body by therapy.

### Technical Problem

An object of the present invention is to provide a composition for prevention or treatment for mild cognitive impairment or dementia.

### Solution to Problem

The present inventors have diligently examined the effect of hydrogen on cognitive impairment and particularly on mild cognitive impairment or dementia. Specifically, the change in mild cognitive impairment was examined in subjects who were diagnosed as mild cognitive impairment and took hydrogen water for 1 year. In this examination, whether the subjects are ApoE ε4 allele carriers (ApoE ε4 genetic polymorphism carriers) or not was determined and whether there is a difference between ApoE ε4 allele carriers and non carriers was also examined.

As a result, the present inventors have found that hydrogen water can prevent or treat mild cognitive impairment or dementia and that it is particularly effective for ApoE ε4 allele carriers, thereby completing the present invention.

Accordingly, the present invention is as follows.
[1] A composition for use in a method for the prevention or treatment for mild cognitive impairment or dementia, comprising or producing molecular or atomic hydrogen as an active ingredient for use in an ApoE ε4 allele carrier.
[2] The composition for use in the prevention or treatment for mild cognitive impairment or dementia according to [1], wherein the composition is a liquid comprising hydrogen.
[3] The composition for use in the prevention or treatment for mild cognitive impairment or dementia according to [2], wherein hydrogen molecules are comprised at saturation.
[4] The composition for use in the prevention or treatment for mild cognitive impairment or dementia according to [1], wherein the composition is hydrogen gas.
[5] The composition for use in the prevention or treatment for mild cognitive impairment or dementia according to [4], wherein the composition comprises the hydrogen gas at a concentration of 1 to 4% (v/v).
[6] The composition for use in the prevention or treatment for mild cognitive impairment or dementia according to [1], wherein the composition is microparticles of a metal or a metal alloy that stores and holds hydrogen or produces hydrogen.
[7] The composition for use in the prevention or treatment for mild cognitive impairment or dementia according to any one of [1] to [6], wherein the composition is a pharmaceutical composition.
[8] The composition for use in the prevention or treatment for mild cognitive impairment or dementia according to any one of [1] to [3], wherein the composition is a health food.
[9] The composition for prevention or treatment for mild cognitive impairment or dementia according to [8], wherein the health food is hydrogen water.
[10] The composition for use in the prevention or treatment for mild cognitive impairment or dementia according to any one of [1] to [9], for improving a cognitive function of a subject with mild cognitive impairment.
[11] The composition for use in the prevention or treatment for mild cognitive impairment or dementia according to any one of [1] to [10], wherein the composition prevents progression from mild cognitive impairment to dementia.
[12] The composition for use in the prevention or treatment for mild cognitive impairment or dementia according to any one of [1] to [9], wherein the composition suppresses the decline of working memory.
[13] The composition for use in the prevention or treatment for mild cognitive impairment or dementia according to [9], wherein the composition improves cognitive function or reduces the risk of mild dementia.

The priority of the present application is based on the contents stated in the description and/or drawings of Japanese patent application No. 2016-138793.

### Advantageous Effects of Invention

The composition for prevention or treatment for mild cognitive impairment or dementia, comprising or producing molecular or atomic hydrogen as an active ingredient according to the present invention makes it possible to prevent mild cognitive impairment or dementia and to treat a subject having mild cognitive impairment or dementia. In accordance with the invention, mild cognitive impairment or dementia can be prevented by administering the composition to or having the composition to be taken by an ApoE ε4 allele carrier (ApoE ε4 genetic polymorphism carrier) found to have a high risk of mild cognitive impairment or dementia.

### Brief Description of Drawings

[Figure 1] Figure 1 illustrates changes in the word recall task score and the total ADAS-cog score before and after the intake of hydrogen water by individual ApoE ε4 genetic polymorphism carriers.
[Figure 2] Figure 2 illustrates one-year changes in the word recall task ability score and the total ADAS-cog score in ApoE ε4 genetic polymorphism carriers and non carriers.
[Figure 3] Figure 3 illustrates an apparatus for administering hydrogen gas to a patient.
[Figure 4] Figure 4 illustrates improvement in spatial working memory by molecular hydrogen in APOE gene defect mice.

### Description of Embodiments

The present invention will be described in detail below.

The present invention is a composition for use in a method for the prevention or treatment for mild cognitive impairment or dementia in an individual who is an ApoE ε4 allele carrier, comprising or producing molecular or atomic hydrogen as an active ingredient.

The composition for prevention or treatment for mild cognitive impairment or dementia, comprising or producing molecular or atomic hydrogen according to the present invention may be a liquid or a gas.

Dementia refers to symptoms and conditions caused by pathological damage to neural cells in the brain and the progression of dementia leads to the decline of the comprehension ability and the judgement ability and interference with social life and daily life. Examples of the dementia according to the present invention include Alzheimer-type dementia, dementia with Lewy bodies, and vascular dementia.

Mild cognitive impairment (MCI) is a prodromal stage for dementia and it is said that neglect of MCI results in continuous decline of the cognitive function and progression to dementia in about 50% of people in 5 years. Mild cognitive impairment causes a problem in one function among cognitive functions (such as memory, decision making, reasoning, and execution), but does not interfere with daily life. Mild cognitive impairment is defined with the following 5 items.
1. The person oneself or a family has complained of memory impairment.
2. Activities of daily living are normal.
3. General cognitive functions are normal.
4. Memory impairment that cannot be explained by only the effect of age and/or the education level is observed.
5. The person is not dementia.

The ApoE ε4 genetic polymorphism of the apolipoprotein E gene has been reported to be a risk factor of mild cognitive impairment and Alzheimer-type dementia. The ApoE protein is a 34 kDa protein consisting of 299 amino acids and there are 3 major isoforms, ApoE2, ApoE3, and ApoE4, which are respectively gene products of 3 alleles ε2, ε3, and ε4. The isoforms ApoE2, ApoE3, and ApoE4 are different from one of them in one amino acid out of the amino acids 112 and 158. ApoE ε2 has Cys/Cys, ApoE ε3 has Cys/Arg, and ApoE ε4 has Arg/Arg.

As seen above, ApoE genotypes include ApoE ε2, ApoE ε3, and ApoE ε4. Among them, increase in ApoE ε4 carried increases the risk of developing mild cognitive impairment and Alzheimer-type dementia in comparison with those carrying only the genotypes APOE ε2 and APOE ε3. APOE genotypes include the ApoE ε2/ApoE ε2 genotype, the ApoE ε2/ApoE ε3 genotype, and the ApoE ε3/ApoE ε3 genotype, which are the genotypes having no ApoE ε4; the ApoE ε2/ApoE ε4 genotype and the ApoE ε3/ApoE ε4 genotype, which are found in 10% of Japanese; and the ApoE ε4/ApoE ε4 genotype, which are found in 2% of Japanese. ApoE ε4 allele carriers (ApoE ε4 genetic polymorphism carriers), that is to say, those having the ApoE ε2/ApoE ε4 heterozygous genotype, the ApoE ε3/ApoE ε4 heterozygous genotype, and the ApoE ε4/ApoE ε4 homozygous genotype (the ApoE ε2/ApoE ε4 heterozygote, the ApoE ε3/ApoE ε4 heterozygote, and the ApoE ε4/ApoE ε4 homozygote) have a higher risk for mild cognitive impairment and Alzheimer-type dementia.

The ApoE gene genotype of a subject can be determined, for example, by collecting blood cells from blood of the subject, extracting DNA, and conducting a usual method for analyzing genetic polymorphism. Examples thereof include methods for analysis by sequence analysis involving direct sequencing by a known method such as the dideoxy method or the Maxam-Gilbert method; methods for hybridization involving use of a probe specific for a genetic polymorphism or a microarray (DNA chip) on which such probes are immobilized; and various methods involving use of a primer specific for a genetic polymorphism. More specific examples include the PCR method, the NASBA method, the LCR method, the SDA method, the LAMP method, methods involving use of restriction fragment length polymorphism (RFLP), denaturing gradient gel electrophoresis (DGGE), methods involving use of chemical cleavage of mismatches (CCM), the primer extension method (the TaqMan (R) method), the PCR-SSCP method, single strand conformation polymorphism (SSCP), the Invader method, the single nucleotide primer method, the SNaPshot method, the MassArray method, the Pyrosequncing method, the SNP-IT method, the BeadArray method, the Scorpion method, and the MADI-TOF/MS method.

The liquid comprising hydrogen molecules is characterized by consisting of an aqueous solution. In the present invention, the liquid comprising hydrogen may be referred to as hydrogen water. The medium composing this aqueous solution may be pure water, ion exchanged water, distilled water, and physiological saline. Furthermore, an agent for removing detrimental free radicals in the body obtained by using pure water, ion exchanged water, or distilled water as the medium may be added to a general aqueous beverage, for example, mineral water, juice, coffee, tea, or the like, to drink the resulting beverage. Here, the beverage includes health foods for drink, foods with function claims, foods for specified health uses, and foods with nutrient function claims. As used herein, the term "foods for specified health uses" refers to foods that are taken for a specific health purpose in diets and have an indication that the health purpose may be achieved by the intake. These beverage may for example have indication that the beverage is used for improving cognitive function or that the beverage is to be used to reduce the risk of having mild dementia.

Hydrogen molecules can be in a dissolved state in water or an aqueous solution for some period of time even at saturation. Such water or an aqueous solution containing hydrogen molecules at saturation can be easily produced by dissolving hydrogen gas in water or an aqueous solution under pressure and then removing the pressure. For example, an aqueous solution may be placed under a hydrogen gas pressure of 0.4 MPa or more for several hours, preferably for 1 to 3 hours. Alternatively, hydrogen water may be produced in a short period of time with an apparatus for producing a large amount of hydrogen water. Hydrogen water in a form of an aqueous solution may be used as drink or used in a form of physiological saline for intravenous administration. In this case, the administration may be performed by administration with a catheter or administration by injection. After injection, hydrogen taken into the body is mostly absorbed by the body and distributed to the whole body through the blood, where it has some effect, and then exhaled with the expiration.

Hydrogen can be dissolved at about 17.5 mL per 1 L of water (about 1.6 ppm, about 0.8 mM) under a hydrogen pressure of 1 atm at room temperature. The liquid composition comprising hydrogen molecules according to the present invention comprises 10 mL or more, preferably 15 mL or more, and particularly preferably 17.5 mL or more of hydrogen molecules per 1 L of aqueous solution. Moreover, the liquid composition comprising hydrogen molecules according to the present invention comprises 0.8 ppm or more, preferably 1 ppm or more, more preferably 1.1 ppm or more, and more preferably 1.2 ppm or more of hydrogen molecules. Moreover, the liquid composition comprising hydrogen molecules according to the present invention comprises 0.1 mM or more, preferably 0.4 mM or more, more preferably 0.6 mM and particularly preferably 0.8 mM or more of hydrogen.

Moreover, the liquid composition comprising hydrogen molecules according to the present invention may comprise oxygen molecules. Oxygen molecules will coexist with hydrogen molecules in an aqueous solution. Even though hydrogen molecules and oxygen molecules are in a mixed state, they do not react immediately and the both can coexist stably. However, when the aqueous solution comprises a large quantity of oxygen molecules, the hydrogen content is preferably less than 4% (v/v) of the total gas to secure safety. Under the environment of use where safety is not a concern, the hydrogen content is preferably a concentration as high as possible. Liquid compositions comprising oxygen may also be used in a form of drink or as intravenous administration in a form of physiological saline. In the case of the administration by injection, such compositions do not cause the state of local oxygen deficiency in the living body and therefore damage the living tissue at a less extent in comparison with compositions without oxygen molecules.

The liquid composition according to the present invention is preferably stored in a container preferably made of a material through which hydrogen cannot penetrate, such as aluminum. Moreover, the composition is preferably stored at a low temperature since more hydrogen is dissolved as the temperature is lowered.

The liquid composition according to the present invention may be taken at 100 to 5000 mL, preferably 150 to 2000 mL, more preferably 150 to 1000 mL, and more preferably 200 to 750 mL per day for a duration of several days to several years, preferably several months to several years, more preferably 6 months to 2 years, and more preferably 6 months to 1 year.

Gas composition comprising hydrogen molecules comprises hydrogen gas. The concentration of the hydrogen gas contained in a gas composition according to the present invention is 1 to 4% (v/v), preferably 2.5 to 3.5% (v/v), and more preferably about 3%. The hydrogen gas content is preferably less than about 4% (v/v) to secure safety, but the hydrogen gas content may be higher under hermetically sealed and safe conditions in which considerations have been made to avoid generation of static electricity. The gas composition comprising hydrogen gas as an active ingredient according to the present invention may further comprise oxygen gas and/or another inert gas. When comprising oxygen gas, the composition is composed of a mixed gas of hydrogen gas and oxygen gas. The oxygen gas is used for breathing. The inert gas may be nitrogen gas, helium gas, argon gas or the like, but nitrogen gas, which is inexpensive, is preferable. The content of this inert gas may be any concentration as determined by a person skilled in the art as long as it is not too much, but 80% (v/v) or less is preferred in consideration of concentration of oxygen gas for breathing. Furthermore, the composition comprising hydrogen gas as an active ingredient according to the present invention may be a mixed gas of hydrogen gas and the air. Such a mixed gas can easily be produced by mixing hydrogen gas with the air as appropriate. Furthermore, the gas composition comprising hydrogen gas as an active ingredient according to the present invention may comprise an anesthetic gas. In this case the gas composition comprising hydrogen gas as an active ingredient is composed of a mixed gas of hydrogen gas and the anesthetic gas. Examples of the anesthetic gas include laughing gas.

The gas composition comprising hydrogen gas as an active ingredient according to the present invention is placed, for example, in a pressure-resistant container such as a gas cylinder. The present invention encompasses containers for containing a gas composition comprising hydrogen gas as an active ingredient.

The gas composition comprising gaseous hydrogen gas as an active ingredient according to the present invention may be taken by aspiration by the subject. The aspiration may be conducted by using an aspiration unit and the gas composition comprising hydrogen gas as an active ingredient may be aspirated from a container comprising the composition via a tube through the aspiration unit. The aspiration unit is not limited, but examples thereof include an inhalation mask and the mask is preferably one that can cover the mouth and the nose of the patient simultaneously. Further examples of the aspiration unit include a hermetic chamber sealed hermetically. The chamber has a size that allows a patient to get in and allows the patient to take by aspiration the gas composition comprising hydrogen gas as an active ingredient according to the present invention by delivering the composition in the chamber with the patient in the chamber. An example of such a chamber is a bed sealed hermetically. A patient can take by aspiration the gas composition comprising hydrogen gas as an active ingredient according to the present invention with lying on a bed.

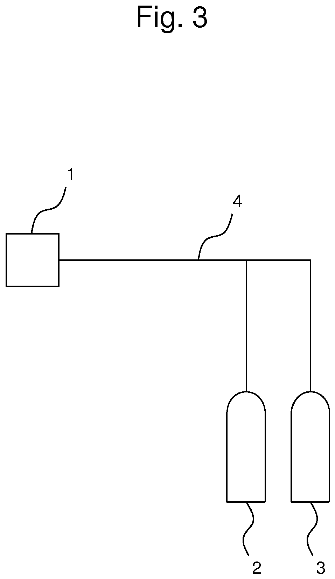

Furthermore, the present invention encompasses containers for containing a gas composition comprising hydrogen gas as an active ingredient and apparatuses for delivering a gas composition comprising hydrogen gas as an active ingredient to a subject, comprising a gas aspiration unit and a delivering tube that delivers the gas in the container to the aspiration unit. For example, the container is a hydrogen gas cylinder. Moreover, examples of the gas aspiration unit include inhalation masks and chambers sealed hermetically, as described above. The apparatuses may further comprise a container containing at least one gas selected from the group consisting of oxygen gas, an inert gas, the air, and an anesthetic gas. In this case, the gas composition comprising hydrogen gas as an active ingredient and at least one gas selected from the group consisting of oxygen gas, an inert gas and the air may be delivered to the gas aspiration unit separately or after mixing. For example, a gas aspiration back containing hydrogen gas is directly connected to an inhalation mask and hydrogen gas may be delivered to this gas aspiration back from a gas cylinder containing hydrogen gas. Figure 3 is a schematic view of an apparatus according to the present invention. The figure comprises an aspiration unit 1; a container 2 containing a therapeutic agent for the acute phase of cerebral infarction comprising hydrogen gas as an active ingredient; a container 3 containing at least one gas selected from the group consisting of oxygen gas, an inert gas, the air, and an anesthetic gas; and a tube 4. The gas is delivered to the gas aspiration unit via the tube and administered to a patient.

The gas composition comprising hydrogen gas as an active ingredient may be administered for 0.1 hours to 5 hours, preferably 0.5 hours to 2 hours, more preferably 1 hour per dose, 1 to 5 doses, preferably 1 to 3 doses, more preferably 2 doses, per day for a few days to several years, preferably several months to several years, more preferably for 6 months to 2 years, more preferably 6 months to 1 year. The aspiration speed for administering hydrogen gas is, for example, several liters, preferably about 6 L per 1 hour.

Furthermore, the composition according to the present invention also comprises a composition comprising a substance that stores and holds hydrogen atoms or produces hydrogen as an active ingredient. The substance that stores and holds hydrogen atoms or produces hydrogen includes microparticles of a metal or a metal alloy that stores and holds hydrogen or produces hydrogen. In the present invention, "hydrogen" includes atomic hydrogen and molecular hydrogen.

Examples of hydrogen storage metals include lithium (Li), magnesium (Mg), calcium (Ca), vanadium (V), and lanthanum (La). In the present invention, hydrogen storage metals or metal alloys include the aforementioned metals that can store hydrogen by combining with hydrogen to become hydrides and alloys thereof. Among these, magnesium or an alloy thereof is preferable. The metal compounds storing hydrogen are referred to as metal hydride compounds: for example, magnesium storing hydrogen is referred to as magnesium hydride (MgH2).

Magnesium hydride reacts with water and produces molecular hydrogen and the remainder turns into Mg(OH)₂, therefore it is safe.

Atomic hydrogen generated from MgH₂ reduces oxides that come in contact (for example, referred to as X in the following chemical equation) by the reaction represented by the following equation.

MgH₂ + 2X → Mg + 2H + 2X → Mg + 2XH

The remaining Mg reacts with H₂O to produce Mg(OH)₂.

The size of the microparticles of the hydrogen storage metal or metal alloy according to the present invention is 0.5 µm to 10 µm, preferably 1 µm to 5 µm, and more preferably 1 µm to 3 µm in average particle size.

The microparticles may be further pulverized (nanoized) and the resulting nanoparticles with a size in nanometer may be used. The size of such nanoparticles is 5 to 500 nm and preferably 10 to 100 nm in average particle size.

Microparticle with different particle sizes according to their purpose may be mixed to have the both effects. Microparticles having a large size produces hydrogen slowly and microparticles having a small size produces hydrogen fast because of their large surface area. Accordingly, mixing microparticles having a small size and microparticles having a large size allows producing hydrogen quickly and continuing the production of hydrogen for a long period of time.

The hydrogen storage metal or metal alloy can be produced by coupling hydrogen with metal and can be produced by a known method. For example, pressure may be increased in the presence of metal and hydrogen. The obtained hydrogen storage metal or metal alloy may be pulverized, for example, by collision of powder particles.

MgH₂ does not react with oxygen in the air in a short time unlike metal Mg and therefore is pulverized by repeating the collision of its particles. A particular size of particles is produced according to the collision speed and the collision frequency. Further pulverization is possible, for example, by a method for causing collision between MgH₂ microparticles and collision of the particles with the wall surface in a hermetically sealed container using an inert gas (e.g., nitrogen) as a carrier gas. In this operation, MgH₂ microparticles having an intended size are obtained by controlling the pressure of carrier gas toward the circumference direction, the flow rate of the gas, and the input of MgH₂ powder. MgH₂ microparticles having smaller sizes, which have higher apparent specific gravity, are moved toward the center by centrifugal force and those having a particle size smaller than an intended size are collected at the center and carried out of the system by the carrier gas. Accordingly, microparticles of the hydrogen storage metal or metal alloy can be further pulverized by providing energy for moving the substance to be pulverized in the circumference direction with a carrier gas and causing collision between particles of the substance of interest or collision between particles and the wall surface or an obstacle by a gas flow with turbulence at many locations.

Molecular hydrogen can be produced by a reaction with water represented by the following equation.

MgH₂ + 2H₂O → Mg(OH)₂ + 2H₂

The microparticles of hydrogen storage metal or metal alloy may be contained in a resin and the present invention also encompasses a composition comprising a resin comprising microparticles of a metal or a metal alloy that stores and holds hydrogen as an active ingredient. The resin comprising microparticles of a metal or a metal alloy that stores and holds hydrogen can produce hydrogen and release it out. Moreover, the present invention is a hydrogen-releasing agent or a hydrogen generant comprising a resin comprising microparticles of a metal or a metal alloy that stores and holds hydrogen.

Mg(OH)₂ itself is not a substance detrimental to the living body, but not preferable to be mixed in drinking water or food. In use of the resin comprising a hydrogen storage metal alloy according to the present invention, Mg(OH)₂ produced when the production of hydrogen is confined in the resin and does not get out of the resin.

Examples of the resin for containing the hydrogen storage metal or metal alloy include polyethylene, polyethylene terephthalate, polypropylene, vinyl chloride, polystyrene, polycarbonate, polyester, polymethyl pentene, styrene, acrylic, nylon, and fluoric resins. Resin to be used is a resin that can be a material of a container for food or used as a material of a film or a sheet for wrapping food.

Examples of methods for including a hydrogen storage metal or metal alloy in a resin include a method involving dissolving or softening the resin with an organic solvent such as methanol, ethanol, isopropyl alcohol, cyclohexane, trichloroethylene, dichloromethane, benzene, ethyl acetate, butyl acetate, acetone, toluene, and xylene or heat to make it in a plastic state and kneading the resin in such a state with the hydrogen storage metal or metal alloy, thereby including the hydrogen storage metal or metal alloy evenly in the resin. The resin comprising a hydrogen storage metal or metal alloy can be produced, for example, by placing a hydrogen storage metal or metal alloy such as MgH₂ in an organic solvent, adding a resin such as polystyrene or polyethylene thereto, dissolving the resulting mixture to make the hydrogen storage metal or metal alloy in a state of being suspended in the dissolved resin, and then evaporating the organic solvent. In such a way, the resin comprising a hydrogen storage metal or metal alloy may be processed into any shape. For example, the resin may be processed, for example, into particles, granules, a sheet, or a film.

Placing the resin comprising a hydrogen storage metal or metal alloy in water or an aqueous solution or under a humid environment makes water pass through and enter the resin to bring water in contact with the hydrogen storage metal or metal alloy and causes release of hydrogen. In this process, the metal or metal alloy such as Mg is held in the resin and the metal or metal alloy is not released from the resin. In this process, the duration of the release of hydrogen from the resin can be regulated by changing the water permeability of the resin and the amount of the hydrogen storage metal or metal alloy contained in the resin. For example, the resin comprising the hydrogen storage metal or metal alloy according to the present invention can release hydrogen for 1 day or more, preferably 3 days or more, more preferably 1 week or more, and particularly preferably 2 weeks or more.

The hydrogen storage metal or metal alloy can release hydrogen at high temperature, e.g., at 270 degrees Celsius or more, but does not release hydrogen at normal temperature and pressure and low humidity under controlled conditions. Moreover, since the resin is hardly permeated by water, the hydrogen storage metal or metal alloy is stored at normal temperature and pressure without releasing and depleting hydrogen for 1 year or more, preferably 3 years or more, more preferably 5 years or more, and particularly preferably 10 years or more.

Moreover, hydrogen-containing water can be produced by pouring water in a container such as a cup and putting the resin comprising hydrogen storage metal or metal alloy according to the invention to produce hydrogen.

The composition comprising a substance that stores and holds hydrogen atoms as an active ingredient may be administered to a subject after producing hydrogen and turning into a state of hydrogen gas or hydrogen water.

The composition for prevention or treatment for mild cognitive impairment or dementia, comprising hydrogen molecules as an active ingredient according to the present invention may be administered to or taken by a subject diagnosed as developing mild cognitive impairment or dementia or may be administered to or taken by a subject at risk of developing mild cognitive impairment or dementia or a subject at risk of developing Alzheimer-type dementia.

In accordance with the invention, the subject at risk of developing mild cognitive impairment or dementia or subject at risk of developing Alzheimer-type dementia is an ApoE ε4 allele carrier (ApoE ε4 genetic polymorphism carrier).

With the composition for prevention or treatment for mild cognitive impairment or dementia, comprising hydrogen molecules as an active ingredient according to the present invention, mild cognitive impairment or dementia can be treated or development of mild cognitive impairment or dementia can be prevented or progression of mild cognitive impairment or dementia can be delayed.

In particular, progression to dementia in a subject with mild cognitive impairment can be prevented.

Whether the composition for prevention or treatment for mild cognitive impairment or dementia, comprising hydrogen molecules as an active ingredient according to the present invention is effective or not can be evaluated based on ADAS-cog (Alzheimer's Disease Assessment Scale-cognitive subscale) (Rosen WG, Mohs RC, Davis KL (1984) A new rating scale for Alzheimer's disease. Am J Psychiatry 141, 1356-1364.), which is a method for evaluating cognitive functions. ADAS-cog evaluates and scores the items: word recall task, spoken language ability, comprehension of spoken language, word-finding difficulty and comprehension, commands, naming task (naming fingers and objects), constructional praxis, ideational praxis, orientation, word recognition, and remembering test instructions. The score ranges from 0 to 70 points (normal to severe).

For example, when the composition for prevention or treatment for mild cognitive impairment or dementia, comprising hydrogen molecules as an active ingredient according to the present invention is administered to or taken by a subject diagnosed as mild cognitive impairment or dementia, the total ADAS-cog score or the word recall task ability score improves (decrease) by one or more point and preferably 2 or more points.

Moreover, the composition comprising hydrogen as an active ingredient according to the present invention can suppress the decline of working memory such as spatial working memory.

Furthermore, the composition comprising hydrogen as an active ingredient according to the present invention can be used for improvement in cognitive functions.

### Examples

The present invention will be specifically described by the following Examples, but the present invention is not limited by these Examples.

### [Example 1] Improvement in cognitive function with the hydrogen water

73 individuals diagnosed as mild cognitive impairment in the Tone project (a project of epidemiology investigation on pre-dementia and depression in 65-year or older residents in Tone-machi, Ibaraki, for the purpose of evaluating feeling, cognitive functions, body functions, and the like, revealing risk factors for dementia, examining possibility of protection against the decline of cognitive functions, and examining possibility of prevention of dementia. Documents: Miyamoto M, Kodama C, Kinoshita T, Yamashita F, Hidaka S, Mizukami K, Kakuma T, Asada T (2009) Dementia and mild cognitive impairment among non-responders to a community survey. J Clin Neurosci 16, 270-276., Bun S, Ikejima C, Kida J, Yoshimura A, Lebowitz AJ, Kakuma T, Asada T (2015) A combination of supplements may reduce the risk of Alzheimer's disease in elderly Japanese with normal cognition. J Alzheimers Dis 45, 15-25.) were classified into 2 groups at random. 35 individuals were given 500 mL per day of hydrogen water (1.2 ppm) and 38 individuals were given 500 mL per day of placebo water. The placebo water was water before the dissolution of hydrogen and was provided in the same package as the hydrogen water. The investigation was conducted as a double-blind test with the placebo water and the hydrogen water provided indistinguishably. The water that was not drunk was recovered to estimate the average daily intake. As a result, the average daily intake was 300 mL for both the hydrogen water and the placebo water.

The AopE ε4 genetic polymorphism was determined by an existing method involving extraction of DNA from blood of the subject and amplification by PCR. The AopE ε4 genetic polymorphism was found in 7 individuals in the hydrogen water group and 6 individuals in the placebo group.

The cognitive function was evaluated based on an existing method of Alzheimer's Disease Assessment Scale (ADAS-cog) (Rosen WG, Mohs RC, Davis KL (1984) A new rating scale for Alzheimer's disease. Am J Psychiatry 141, 1356-1364). Word recall task, spoken language ability, comprehension of spoken language, word-finding difficulty and comprehension, commands, naming task (naming fingers and objects), constructional praxis, ideational praxis, orientation, word recognition, and remembering test instructions were scored according to the existing method. In this method, increase in the score means aggravation.

The hydrogen water or placebo water was given for 1 year. The change in ADAS-cog before to after the intake is set forth in Table 1. Furthermore, analysis of statistical difference was conducted by statistical analysis by t-test (Student t-test) to determine p-values and changes with p < 0.05 were determined as statistically significant changes as generally accepted.

**[Table 1]**

| Average | | | Word Recall Task | Spoken language Ability | Comprehension of Spoken Languaqe | Word-Finding Difficulty and Comprehension | Commands | Naming Task | Constructional Praxis | Ideational Praxis | Orientation | Word Recognition | Remembering Test Instructions | ADAS total |
|---|---|---|---|---|---|---|---|---|---|---|---|---|---|---|
| ADAS-cog (Control) | n=38 | Before | 4.7 | 0.0 | 0.2 | 0.1 | 0.2 | 0.0 | 0.2 | 0.0 | 0.0 | 2.5 | 0.1 | 7.89 |
| | | After | 4.1 | 0.0 | 0.2 | 0.0 | 0.2 | 0.0 | 0.1 | 0.0 | 0.1 | 1.7 | 0.3 | 6.73 |
| ADAS-cog (Intake of hydrogen water) | n=35 | Before | 4.8 | 0.0 | 0.1 | 0.0 | 0.3 | 0.0 | 0.3 | 0.0 | 0.0 | 2.3 | 0.2 | 8.04 |
| | | After | 4.2 | 0.0 | 0.1 | 0.0 | 0.1 | 0.0 | 0.2 | 0.0 | 0.0 | 1.2 | 0.2 | 6.00 |
| | | | 0.914 | 1.000 | 0.641 | 0.673 | 0.238 | 0.341 | 0.547 | 0.301 | 0.197 | 0.523 | 0.299 | 0.243 |

| ApoE ε4 carriers average | | | Word Recall Task | Spoken language Ability | Comprehension of Spoken Language | Word-Finding Difficulty and Comprehension | Commands | Naming Task | Constructional Praxis | Ideational Praxis | Orientation | Word Recognition | Remembering Test Instructions | ADAS total |
|---|---|---|---|---|---|---|---|---|---|---|---|---|---|---|
| ADAS-cog (Control) | n=6 | Before | 4.7 | 0.0 | 0.0 | 0.0 | 0.2 | 0.0 | 0.0 | 0.0 | 0.0 | 2.4 | 0.0 | 7.32 |
| | | After | 4.6 | 0.0 | 0.3 | 0.0 | 0.2 | 0.0 | 0.0 | 0.0 | 0.0 | 1.6 | 0.3 | 7.03 |
| ADAS-oog (Intake of hydrogen water) | n=7 | Before | 5.6 | 0.0 | 0.0 | 0.0 | 0.3 | 0.0 | 0.3 | 0.0 | 0.0 | 2.6 | 0.4 | 9.22 |
| | | After | 4.3 | 0.0 | 0.1 | 0.0 | 0.1 | 0.0 | 0.1 | 0.0 | 0.1 | 1.2 | 0.4 | 6.50 |
| | | Two-side test | 0.039 | 1.000 | 0.590 | 1.000 | 0.624 | 1.000 | 0.377 | 1.000 | 0.377 | 0.545 | 0.642 | 0.040 |

| ApoE ε4 non carriers average | | | Word Recall Task | Spoken language Ability | Comprehension of Spoken Language | Word-Finding Difficulty and Comprehension | Commands | Naming Task | Constructional Praxis | Ideational Praxis | Orientation | Word Recognition | Remembering Test Instructions | ADAS total |
|---|---|---|---|---|---|---|---|---|---|---|---|---|---|---|
| ADAS-cog (Control) | n=32 | Before | 4.7 | 0.0 | 0.2 | 0.1 | 0.2 | 0.0 | 0.2 | 0.0 | 0.0 | 2.5 | 0.2 | 8.00 |
| | | After | 4.0 | 0.0 | 0.2 | 0.0 | 0.2 | 0.0 | 0.1 | 0.0 | 0.1 | 1.7 | 0.3 | 6.68 |
| ADAS-cog (Intake of hydrogen water) | n=28 | Before | 4.6 | 0.0 | 0.1 | 0.0 | 0.3 | 0.0 | 0.3 | 0.0 | 0.0 | 2.3 | 0.1 | 7.75 |
| | | After | 4.1 | 0.0 | 0.1 | 0.0 | 0.1 | 0.0 | 0.2 | 0.0 | 0.0 | 1.2 | 0.1 | 5.88 |
| | | Two-side test | 0.530 | 1.000 | 0.729 | 0.681 | 0.284 | 0.354 | 0.712 | 0.289 | 0.052 | 0.653 | 0.354 | 0.538 |

Overall, there was a tendency for hydrogen water to have larger improvement effect with the placebo group having an average improvement of 1.06 points and the hydrogen water group having an average improvement of 2.04 points. In the non-ApoE ε4 genetic polymorphism carrier group, there was a tendency for hydrogen water to have larger improvement effect in the hydrogen water group, with the placebo group having an average improvement of 1.32 points and the hydrogen water group having an average improvement of 1.87 points.

In the ApoE ε4 genetic polymorphism carriers, the differences in the total word recall task and total ADAS-cog scores after one year were compared. The improvement in the word recall task ability was 0.1 points in the placebo group and 1.3 points in the hydrogen water group. The improvement in the total ADAS-cog score was 0.29 points in the placebo group and 2.72 points in the hydrogen water group. These are statistically significant changes with p = 0.039 and p = 0.04, respectively.

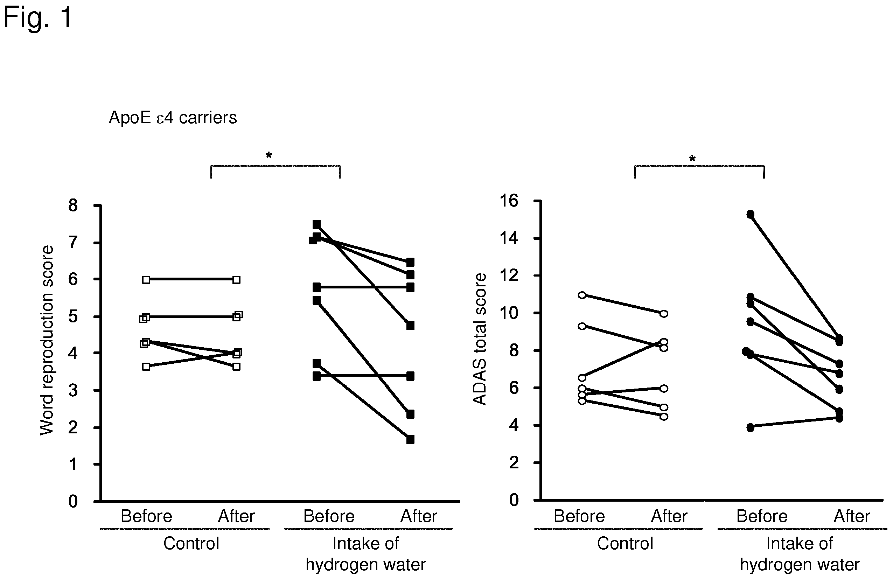

Figure 1 illustrates the change in score on the word recall task ability and total ADAS-cog score by individual ApoE ε4 genetic polymorphism carriers. As illustrated in the figure, intake of hydrogen water leads to decrease in the word recall task ability score and the total ADAS-cog score in all of the ApoE ε4 genetic polymorphism carriers, which has revealed improvement in both the word recall task ability and total cognitive functions.

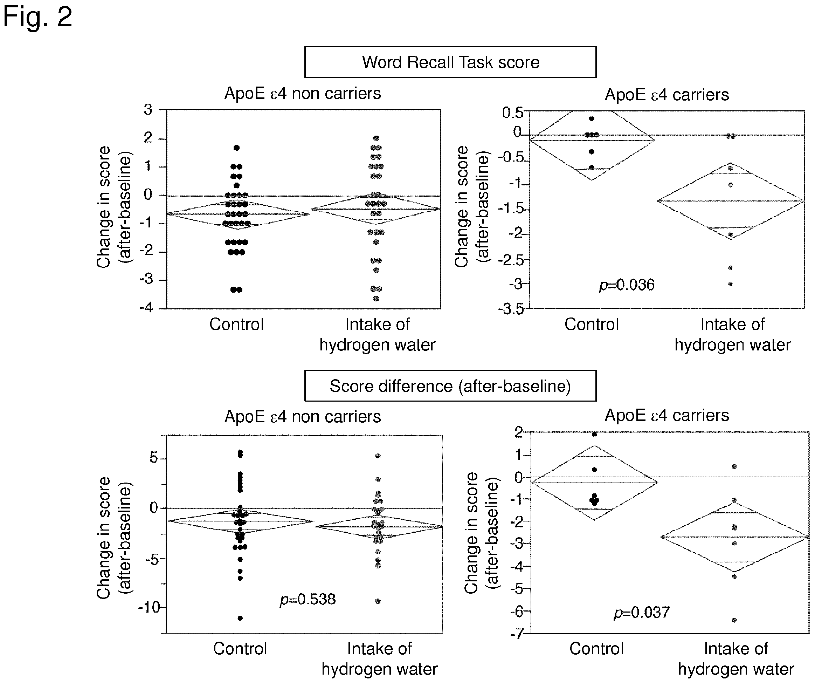

Figure 2 illustrates the one-year change of the score on the word recall task ability and the total ADAS-cog score in ApoE ε4 genetic polymorphism carriers and non carriers for 95% (upper vertex), 75% (upper bar), mean (middle bar), 25% (lower bar), 5% (lower vertex).

The effect of hydrogen water on the ApoE ε4 genetic polymorphism carriers was apparent in the both.

### [Example 2] Spatial working memory in APOE deficient mice

In human, it is known that the ApoE gene has alleles and ApoE4 is a risk for arteriosclerosis and Alzheimer-type dementia. In mice, the APOE gene deficiency is similarly known as model animals for dementia and arteriosclerosis (respectively, Ohsawa I. et al., J Neurosci. 2008;28:6239-6249, Ohsawa I. et al., Biochem Biophys Res Commun. 2008;377:1195-1198). The spatial working memory was measured using dementia model mice. Furthermore, it was examined using mice whether methods of intake of molecular hydrogen (H2) other than hydrogen water, such as hydrogen gas and hydrogen generating materials, are also effective.

### Subject mouse

The wild type mice used were BALB/cCrSlc and the APOE-gene deficient mice used were BALB/c.KOR/StmSlc-Apoe^{Sh1}. This murine line is spontaneous ApoE deficient mice (http://www.jslc.co.jp/inform/shl_basic_data.pdf#search=%27C.KOR%2FStmSlcApoe+shl%2 7).

Molecular hydrogen was administered to 6 mice aged 8 weeks in each group and spatial working memory of the animals was measured at 10-month old.

### Measurement of spatial working memory

The cognitive function was measured by the Y maze test (Conrad CD.et al., Brain Res. 1997; 759:76-83). Its principle is as follows. Animals placed in the center of 3 arms in the Y-shape start exploratory behavior after a while. Normal animals rarely return to the arm once explored and explore different arms in turn with the help of marks seen out of the maze. However, model animals with declined space recognition and/or memory go to the same place many times and enter the arm that the animal entered the last time again. In the Y maze test, the order of the arms that an animal entered during a certain period of time is measured and the percentage of the number of times (percent alternation) when the mouse entered all different arms in a consecutive raw of three times as an indicator of space recognition and memory.

### Method for administering molecular hydrogen

### (1) Free intake of hydrogen water

The animals were allowed to take saturated hydrogen water freely from a glass container filled with the hydrogen water and having a drinker containing 2 ball bearings. The 2 ball bearings in the drinker prevent hydrogen from getting away. As a control, the same container was filled with water before dissolution of hydrogen and the animals were allowed to take freely. The hydrogen water and the control water were changed with fresh water five times a week. (2) Free intake of MgH₂ containing diet

MgH₂ reacts with water to produce hydrogen (MgH₂ + 2H₂O → 2H₂ + Mg(OH)₂). Powder feed containing 0.1 g of MgH₂ in 1 kg of MF powder feed (a product manufactured by Oriental Yeast Co., ltd.) was produced and the animals were allowed to take the feed freely. This is based on the calculation indicating that hydrogen equal to 10 times of hydrogen provided from hydrogen water can be taken, in an assumption that 100% of the hydrogen is maintained as MgH₂ and 100% of the hydrogen is generated in the murine body. The control feed was feed containing 0.22 g of Mg(OH)₂ per 1 kg of MF powder feed. The feed was changed with fresh feed five times a week.

### (3) Inhalation of hydrogen gas

Mice were placed and left for 1 hour five times a week in a chamber containing 2% hydrogen gas and 98% air to make them inhale hydrogen gas for 1 hour. The control mice were placed for 1 hour in a chamber containing the air five times a week.

### Result of analysis

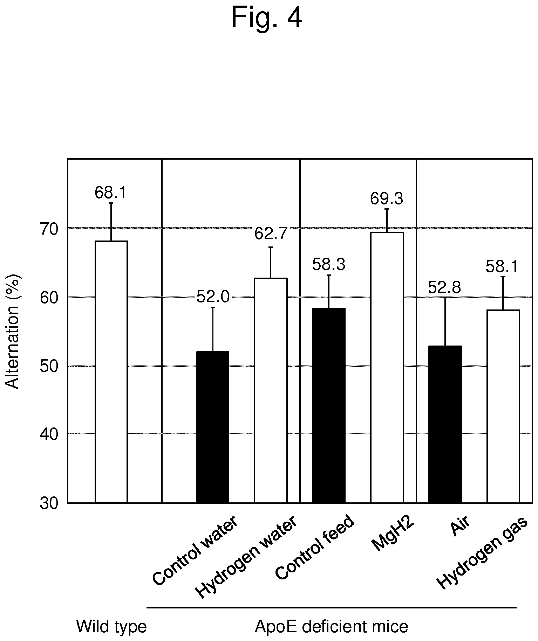

Mice were allowed to take hydrogen or a control of interest from the age of 8 weeks and examined for spatial working memory by the Y maze 250 days later (at 10-month old) (Figure 4). In the APOE gene deficient mice, spatial working memory was declined in comparison with the wild type mice.

In the APOE gene deficient mice that had taken hydrogen, regardless of the method of intake, including drinking hydrogen water, MgH₂ intake, and inhalation of hydrogen gas, the decline of spatial working memory was decreased. This has indicated that hydrogen is effective in suppressing the decline of spatial working memory.

### Industrial Applicability

The composition comprising hydrogen molecules as an active ingredient according to the present invention is available in prevention or treatment of mild cognitive impairment.

### Reference Signs List

- 1: Gas aspiration unit
- 2: Container containing hydrogen gas
- 3: Container containing at least one gas selected from the group consisting of oxygen gas, an inert gas, the air, and an anesthetic gas
- 4: Tube

## Claims

1. A composition for use in a method for the prevention or treatment of mild cognitive impairment or dementia in an individual who is an ApoE ε4 allele carrier, wherein the composition comprises or produces molecular or atomic hydrogen as an active ingredient.

2. The composition for the use of claim 1, wherein the composition is a liquid comprising hydrogen.

3. The composition for the use of claim 2, wherein hydrogen molecules are comprised at saturation.

4. The composition for the use of claim 1, wherein the composition is hydrogen gas.

5. The composition for the use of claim 4, wherein the composition comprises the hydrogen gas at a concentration of 1 to 4% (v/v).

6. The composition for the use of claim 1, wherein the composition is microparticles of a metal or a metal alloy that occludes and holds hydrogen or produces hydrogen.

7. The composition for the use of any one of claims 1 to 6, wherein the composition is a pharmaceutical composition.

8. The composition for the use of any one of claims 1 to 3, wherein the composition is a health food.

9. The composition for the use of claim 8, wherein the health food is hydrogen water.

10. The composition for the use of any one of claims 1 to 9, wherein the composition is for use for improving a cognitive function of a subject with mild cognitive impairment.

11. The composition for the use of any one of claims 1 to 10, wherein the composition is for use to prevent or delay progression from mild cognitive impairment to dementia.

12. The composition for the use of any one of claims 1 to 9, wherein the composition is for use to suppress the decline of working memory.

13. The composition for the use of claim 9, wherein the composition is for use for improving cognitive function or reducing the risk of mild dementia.

## Patentansprüche

1. Zusammensetzung zur Verwendung in einem Verfahren zur Prävention oder Behandlung einer milden kognitiven Beeinträchtigung oder von Demenz in einem Individuum, das ein Träger des ApoE-ε4-Allels ist, wobei die Zusammensetzung molekularen oder atomaren Wasserstoff als Wirkstoff umfasst oder produziert.

2. Zusammensetzung zur Verwendung nach Anspruch 1, wobei die Zusammensetzung eine Flüssigkeit, umfassend Wasserstoff, ist.

3. Zusammensetzung zur Verwendung nach Anspruch 2, wobei Wasserstoffmoleküle bei Sättigung umfasst sind.

4. Zusammensetzung zur Verwendung nach Anspruch 1, wobei die Zusammensetzung Wasserstoffgas ist.

5. Zusammensetzung zur Verwendung nach Anspruch 4, wobei die Zusammensetzung das Wasserstoffgas in einer Konzentration von 1 bis 4 Vol.-% umfasst.

6. Zusammensetzung zur Verwendung nach Anspruch 1, wobei die Zusammensetzung Mikropartikel eines Metalls oder einer Metalllegierung ist, die Wasserstoff einschließen und halten oder Wasserstoff produzieren.

7. Zusammensetzung zur Verwendung nach einem der Ansprüche 1 bis 6, wobei die Zusammensetzung eine pharmazeutische Zusammensetzung ist.

8. Zusammensetzung zur Verwendung nach einem der Ansprüche 1 bis 3, wobei die Zusammensetzung Reformkost ist.

9. Zusammensetzung zur Verwendung nach Anspruch 8, wobei die Reformkost Wasserstoffwasser ist.

10. Zusammensetzung zur Verwendung nach einem der Ansprüche 1 bis 9, wobei die Zusammensetzung zur Verwendung zum Verbessern einer kognitiven Funktion eines Individuums mit milder kognitiver Beeinträchtigung dient.

11. Zusammensetzung zur Verwendung nach einem der Ansprüche 1 bis 10, wobei die Zusammensetzung zur Verwendung zur Prävention oder Verzögerung des Fortschreitens von milder kognitiver Beeinträchtigung zu Demenz dient.

12. Zusammensetzung zur Verwendung nach einem der Ansprüche 1 bis 9, wobei die Zusammensetzung zur Verwendung zum Unterdrücken des Verfalls des Arbeitsgedächtnisses dient.

13. Zusammensetzung zur Verwendung nach Anspruch 9, wobei die Zusammensetzung zur Verwendung zum Verbessern der kognitiven Funktion oder zum Reduzieren des Risikos für milde Demenz dient.

## Revendications

1. Composition pour une utilisation dans un procédé pour la prévention ou le traitement d'une déficience cognitive légère ou d'une démence chez un individu qui est porteur d'allèle ε4 d'ApoE, dans laquelle la composition comprend ou produit de l'hydrogène moléculaire ou atomique en tant que principe actif.

2. Composition pour l'utilisation selon la revendication 1, dans laquelle la composition est un liquide, comprenant de l'hydrogène.

3. Composition pour l'utilisation selon la revendication 2, dans laquelle les molécules d'hydrogène sont contenues à saturation.

4. Composition pour l'utilisation selon la revendication 1, dans laquelle la composition est de l'hydrogène gazeux.

5. Composition pour l'utilisation selon la revendication 4, dans laquelle la composition comprend l'hydrogène gazeux à une concentration comprise entre 1 et 4 % (v/v).

6. Composition pour l'utilisation selon la revendication 1, dans laquelle la composition est constituée de microparticules d'un métal ou d'un alliage métallique qui boque et retient de l'hydrogène ou produit de l'hydrogène.

7. Composition pour l'utilisation selon l'une quelconque des revendications 1 à 6, dans laquelle la composition est une composition pharmaceutique.

8. Composition pour l'utilisation selon l'une quelconque des revendications 1 à 3, dans laquelle la composition est un aliment diététique.

9. Composition pour l'utilisation selon la revendication 8, dans laquelle l'aliment diététique est de l'eau hydrogénée.

10. Composition pour l'utilisation selon l'une quelconque des revendications 1 à 9, dans laquelle la composition est pour une utilisation afin d'améliorer une fonction cognitive d'un sujet atteint d'une déficience cognitive légère.

11. Composition pour l'utilisation selon l'une quelconque des revendications 1 à 10, dans laquelle la composition est à utiliser pour prévenir ou retarder une progression d'une déficience cognitive légère à une démence.

12. Composition pour l'utilisation selon l'une quelconque des revendications 1 à 9, dans laquelle la composition est pour une utilisation afin de supprimer le déclin de mémoire de travail.

13. Composition pour l'utilisation selon la revendication 9, dans laquelle la composition est pour une utilisation afin d'améliorer une fonction cognitive ou réduire le risque de démence légère.
